# EUROPÄISCHE PATENTSCHRIFT

(11) **EP 0 736 084 B1**
(45) Veröffentlichungstag und Bekanntmachung des Hinweises auf die Patenterteilung: **09.09.1998**
(21) Anmeldenummer: 95905065.9
(22) Anmeldetag: 14.12.1994
(51) Int. Cl.: C11D 3/386, C11D 17/00, C11D 3/12, C11D 3/02, C12N 9/98

(54) **SILBERKORROSIONSSCHUTZMITTELHALTIGE ENZYMZUBEREITUNG**
ENZYME PREPARATION CONTAINING A CORROSION PROTECTING AGENT FOR SILVER
PREPARATION A BASE D'ENZYME RENFERMANT UN INHIBITEUR DE CORROSION DE L'ARGENT

(30) Priorität: 23.12.1993 DE 4344215
(43) Veröffentlichungstag der Anmeldung: 09.10.1996
(73) Patentinhaber: Henkel Kommanditgesellschaft auf Aktien, 40191 Düsseldorf (DE)
(72) Erfinder: PAATZ, Kathleen, D-40593 Düsseldorf (DE); RÄHSE, Wilfried, D-40598 Düsseldorf (DE); HÄRER, Jürgen, D-40597 Düsseldorf (DE); PICHLER, Werner, A-6250 Kundl (AT); BURG, Birgit, D-46519 Alpen (DE)
(86) Internationale Anmeldenummer: EP9404152
(87) Internationale Veröffentlichungsnummer: WO9517493

(56) Entgegenhaltungen:
- EP-A- 0 290 223
- EP-A- 0 415 652
- WO-A-92/11347
- WO-A-94/26859
- WO-A-94/26860
- DE-A- 4 128 672

## Beschreibung

Die Erfindung betrifft ein Enzymgranulat, welches einen Silberkorrosionsinhibitor enthält, ein Verfahren zu seiner Herstellung und die Verwendung des Granulats in festen Reinigungsmitteln, insbesondere zur maschinellen Geschirreinigung.

Enzyme, insbesondere Proteasen, finden ausgedehnte Verwendung in Wasch-, Waschhilfs- und Reinigungsmitteln. Üblicherweise kommen die Enzyme dabei nicht als Konzentrate, sondern in Mischungen mit einem Verdünnungs- und Trägermaterial zum Einsatz. Mischt man solche Enzymzubereitungen üblichen Wasch- oder Reinigungsmitteln bei, so kann beim Lagern ein erheblicher Abbau der Enzymaktivität eintreten, insbesondere wenn bleichaktive Verbindungen zugegen sind. Das Aufbringen der Enzyme auf Trägersalze unter gleichzeitiger Granulation gemäß der deutschen Offenlegungsschrift DT 16 17 190 beziehungsweise durch Aufkleben mit nichtionischen Tensiden gemäß der deutschen Offenlegungsschrift DT 16 17 118 oder wäßrigen Lösungen von Celluloseethern gemäß der deutschen Offenlegungschrift DT 17 87 568 führt nicht zu einer nennenswerten Verbesserung der Lagerstabilität, da sich die empfindlichen Enzyme in solchen Aufmischungen in der Regel auf der Oberfläche der Trägersubstanz befinden. Zwar kann die Lagerstabilität der Enzyme wesentlich erhöht werden, wenn man die Enzyme mit dem Trägermaterial umhüllt beziehungsweise in dieses einbettet und anschließend durch Extrudieren, Pressen und Marumerisieren in die gewünschte Partikelform überführt, wie zum Beispiel in der deutschen Patentschrift DE 16 17 232, der deutschen Offenlegungsschrift DT 20 32 768, und den deutschen Auslegeschriften DE 21 37 042 und DE 21 37 043 beschrieben. Derartige Enzymzubereitungen besitzen jedoch nur mangelhafte Löslichkeitseigenschaften. Die ungelösten Partikel können sich im zu reinigenden Gut verfangen und dieses verunreinigen bzw. sie werden ungenutzt in das Abwasser überführt. Aus der deutschen Offenlegungsschrift DT 18 03 099 bekannte Einbettungsmittel, die aus einem Gemisch fester Säuren beziehungsweise saurer Salze und Carbonaten beziehungsweise Bicarbonaten bestehen und bei Wasserzusatz zerfallen, verbessern zwar das Lösungsvermögen, sind aber ihrerseits sehr empfindlich gegen Feuchtigkeit und erfordern daher zusätzliche Schutzmaßnahmen.

Aus der europäischen Patentschrift EP 168 526 sind Enzymgranulate bekannt, die in Wasser quellfähige Stärke, Zeolith und wasserlösliches Granulierhilfsmittel enthalten. In diesem Dokument wird ein Herstellungsverfahren für derartige Formulierungen vorgeschlagen, das im wesentlichen darin besteht, eine von unlöslichen Bestandteilen befreite Fermenterlösung aufzukonzentrieren, mit den genannten Zuschlagstoffen zu versetzen. das entstandene Gemisch zu granulieren und gegebenenfalls das Granulat mit filmbildenden Polymeren und Farbstoffen zu umhüllen. Das Verfahren mit dem dort vorgeschlagenen Zuschlagstoffgemisch wird vorteilhaft mit Fermentationslösungen durchgeführt, die auf einen relativ hohen Trockensubstanzgehalt, beispielsweise 55 Gew.-%, aufkonzentriert worden sind. Außerdem weisen die derart hergestellten Granulate eine so hohe Lösungs- beziehungsweise Zerfallsgeschwindigkeit unter Einsatzbedingungen auf, daß die Granulate teilweise schon bei der Lagerung relativ rasch zerfallen und die Enzyme desaktiviert werden.

Aus der internationalen Patentanmeldung WO 92/11347 sind Enzymgranulate zum Einsatz in körnigen Wasch- und Reinigungsmitteln bekannt, die 2 Gew.-% bis 20 Gew.-% Enzym, 10 Gew.-% bis 50 Gew.-% quellfähige Stärke, 5 Gew.-% bis 50 Gew.-% wasserlösliches organisches Polymer als Granulierhilfsmittel, 10 Gew.-% bis 35 Gew.-% Getreidemehl und 3 Gew.-% bis 12 Gew.-% Wasser enthalten. Durch ein derartiges Zuschlagstoffgemisch wird die Enzymverarbeitung ohne größere Aktivitätsverluste möglich und auch die Lagerbeständigkeit der Enzyme in den Granulaten ist zufriedenstellend.

Die europäischen Patentanmeldungen EP-A-0 415 652 und EP-A-0 290 223 offenbaren enzymhaltige Bleichmittel, in denen das Enzym in Form umhüllter Granulate enthalten ist, wobei die Umhüllungsschicht einen Wirkstoff, insbesondere ein Übergangsmetallsalz wie CuSO₄, FeSO₄, CoSO₄ oder NiSO₄, aufweist, welcher das Enzym vor oxidativer Inaktivierung schützt.

Ein insbesondere bei Reinigungsmitteln für das maschinelle Geschirrspülen weiteres Problem ist die Notwendigkeit, in derartige Mittel Korrosionsinhibitoren für Tafelsilber einzuarbeiten, wenn die Mittel die in neuerer Zeit üblichen Bleich- beziehungsweise Oxidationsmittel auf Sauerstoffbasis enthalten. Silber kann beim Reinigen mit schwefelhaltigen Substanzen, die im Spülwasser gelöst beziehungsweise dispergiert sind, reagieren, denn bei der Reinigung von Geschirr in Haushaltsgeschirrspülmaschinen werden Speisereste und damit unter anderem auch Senf, Erbsen, Ei und sonstige schwefelhaltige Verbindungen wie Mercaptoaminosäuren in die Spülflotte eingebracht. Auch die während des maschinellen Spülens viel höheren Temperaturen und die längeren Kontaktzeiten mit den schwefelhaltigen Speiseresten begünstigen im Vergleich zum manuellen Spülen das Anlaufen von Silber. Durch den intensiven Reinigungsprozeß in der Spülmaschine wird die Silberoberfläche außerdem vollständig entfettet und dadurch empfindlicher gegenüber chemischen Einflüssen.

Das Problem des Silberanlaufens wird insbesondere dann akut, wenn alternativ zu den schwefelhaltige Substanzen oxidativ "entschärfenden" Aktivchlorverbindungen Aktivsauerstoffverbindungen, wie beispielsweise Natriumperborat oder Natriumpercarbonat eingesetzt wurden, welche zur Beseitigung bleichbarer Anschmutzungen, wie beispielsweise Teeflecken/Teebeläge, Kaffeerückstände, Farbstoffe aus Gemüse, Lippenstiftreste und dergleichen dienen.

Diese Aktivsauerstoffverbindungen werden, in der Regel zusammen mit Bleichaktivatoren, vor allem in modernen niederalkalischen maschinellen Spülmitteln der neuen Reinigergeneration eingesetzt. Diese Mittel bestehen im allgemeinen aus den folgenden Funktionsbausteinen: Builderkomponente (Komplexbildner/Dispergiermittel), Alkaliträger, Bleichsystem (Bleichmittel + Bleichaktivator), Enzym und Tensid. Unter den bei Einsatz derartig aufgebauter Mittel auftretenden Spülbedingungen bilden sich in Gegenwart von Silber nicht nur sulfidische, sondern durch den oxidierenden Angriff der intermediär gebildeten Peroxide beziehungsweise des Aktivsauerstoffs auch oxidische Beläge auf den Silberoberflächen.

Die Vermeidung der Silberkorrosion, das heißt die Bildung sulfidischer, oxidischer oder chloridischer Beläge auf Silber ist das Thema zahlreicher Veröffentlichungen. Aus der britischen Patentschrift GB 1 131 738 sind alkalische Geschirrspülmittel bekannt, die als Korrosionsinhibitor für Silber Benzotriazole enthalten. In der US-amerikanischen Patentschrift US 3 549 539 werden stark alkalische, maschinell anwendbare Geschirreinigungsmittel beschrieben, die als Oxidationsmittel unter anderem Perborat mit einem organischen Bleichaktivator enthalten können. Als Anlaufverhinderungsmittel werden Zusätze ebenfalls von Benzotriazol und auch Eisen(III)-chlorid empfohlen. In den europäischen Patentschriften EP 135 226 und EP 135 227 werden schwach alkalische maschinell anwendbare Geschirrspülmittel mit einem Gehalt an Peroxyverbindungen und Aktivatoren beschrieben, die als Silberschutzmittel Benzotriazole und Fettsäuren enthalten können. Schließlich ist aus der deutschen Offenlegungsschrift DE 41 28 672 bekannt, daß Peroxyverbindungen, die durch Zusatz bekannter organischer Bleichaktivatoren aktiviert werden, in stark alkalischen Reinigungsmitteln das Anlaufen von Silberteilen verhindern. Die nicht vorveröffentlichten deutschen Patentanmeldungen DE 43 25 922.7 = WO-A-94/26859 beziehungsweise DE 43 15 397.6 = WO-A-94/26860 beschreiben die Verwendung von Titan-, Zirkonium-, Hafnium-, Vanadium-, Cobalt- oder Cersalzen und/oder - komplexen, in denen die Metalle in einer der Oxidationsstufen II, III, IV, V oder VI vorliegen, beziehungsweise von Mangan(II)-salzen oder -komplexen zur Verhinderung der Silberkorrosion.

Auch wenn die genannten Reinigungsmittel relativ wenig komplex zusammengesetzt sind, ist man dennoch bestrebt, im Rahmen ihrer Herstellung Komponenten einzusetzen, die mehr als einen der oben aufgeführten Wirkstoffe (Builder, Alkaliträger, Bleichsystem, Enzym, Tensid, Silberkorrosionsinhibitor) enthalten.

Es bestand daher die Aufgabe, ein Verfahren zu entwickeln, welches in der Lage ist, auf möglichst einfachem Wege ein enzymhaltiges Granulat, das zusätzlich Silberkorrosionsschutzmittel, beispielsweise in Form bestimmter anorganischer Salze enthält, herzustellen, in dem die genannten Komponenten, insbesondere das Enzym, lagerstabil sind.

Die Erfindung betrifft demgemäß ein für die Einarbeitung in insbesondere teilchenförmige Reinigungsmittel geeignetes Enzymgranulat, enthaltend im Kern Enzym und anorganisches und/oder organisches Trägermaterial sowie gegebenenfalls Granulierhilfsmittel, auf den eine gleichmäßige Umhüllungsschicht aufgebracht ist, welches dadurch gekennzeichnet ist, daß die Umhüllungsschicht einen insbesondere anorganischen Silberkorrosionsinhibitor enthält, welcher nicht CoSO₄ ist. Dabei sind Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vor-liegen, bevorzugt.

Das Wort "Korrosion" ist in seiner weitesten in der Chemie gebräuchlichen Bedeutung auszulegen, insbesondere soll hier "Korrosion" für jede visuell gerade noch erkennbare Veränderung einer Metalloberfläche, hier Silber, stehen, sei es zum Beispiel eine punktuelle Verfärbung, sei es zum Beispiel ein großflächiges Anlaufen.

Die als bevorzugte Silberkorrosionsinhibitoren zu verwendenden Metallsalze beziehungsweise Metallkomplexe sollen zumindest teilweise in Wasser löslich sein. Die zur Salzbildung geeigneten Gegenionen umfassen alle üblichen ein-, zwei-, oder dreifach negativ geladenen anorganischen Anionen, zum Beispiel Oxid, Sulfat, Nitrat, Fluorid, aber auch organische Anionen wie zum Beispiel Stearat.

Metallkomplexe im Sinne der Erfindung sind Verbindungen, die aus einem Zentralatom und einem oder mehreren Liganden sowie gegebenenfalls zusätzlich einem oder mehreren der obengenannten Anionen bestehen. Das Zentralatom ist dabei eines der obengenannten Metalle in einer der obengenannten Oxidationsstufen. Die Liganden sind neutrale Moleküle oder Anionen, die ein- oder mehrzähnig sein können; der Begriff "Ligand" im Sinne der Erfindung ist beispielsweise in "Römpp Chemie Lexikon, Georg Thieme Verlag Stuttgar/New York, 9. Auflage, 1990, Seite 2507" näher erläutert. Ergänzen sich in einem Metallkomplex die Ladung des Zentralatoms und die Ladung des/der Liganden nicht auf Null, so sorgt, je nachdem, ob ein kationischer oder ein anionischer Ladungsüberschuß vorliegt, entweder eines oder mehrere der obengenannten Anionen oder ein oder mehrere Kationen, zum Beispiel Natrium-, Kalium-, Ammoniumionen, für den Ladungsausgleich.

Bevorzugte Metallsalze und/oder Metallkomplexe sind MnSO₄, V₂O₅, V₂O₄, VO₂, TiOSO₄, K₂TiF₆, K₂ZrF₆, Co(NO₃)₂, Ce(NO₃)₃ sowie deren Gemische. Bei diesen Metallsalzen beziehungsweise Metallkomplexen handelt es sich um handelsübliche Substanzen, die zum Zwecke des Silberkorrosions-Schutzes ohne vorherige Reinigung eingesetzt werden können. So ist zum Beispiel das aus der SO₃-Herstellung (Kontaktverfahren) bekannte Gemisch aus fünf- und vierwertigem Vanadium (V₂O₅, VO₂, V₂O₄) geeignet, ebenso wie das durch Verdünnen einer Ti(SO₄)₂-Lösung mit Wasser entstehende Titanylsulfat TiOSO₄.

Vorzugsweise ist im fertigen Enzymgranulat 40 Gew.-% bis 95 Gew.-%, insbesondere 50 Gew.-% bis 85 Gew.-% enzymhaltiger Kern und 5 Gew.-% bis 60 Gew.-%, insbesondere 15 Gew.-% bis 50 Gew.-% Umhüllungsschicht enthalten. Dabei kann die gesamte Umhüllungsschicht aus dem Silberkorrosionsinhibitor bestehen. In einer bevorzugten Ausführungsform des erfindungsgemäßen Enzymgranulats besteht die Umhüllungsschicht des enzymhaltigen Kerns jedoch aus einem Umhüllungssystem, welches 45 Gew.-% bis 90 Gew.-%, insbesondere 50 Gew.-% bis 80 Gew.-% Silberkorrosionsinhibitor, bis zu 25 Gew.-%, insbesondere 5 Gew.-% bis 20 Gew.-% feinteiliges anorganisches Pigment, 5 Gew.-% bis 20 Gew.-% eines bei Raumtemperatur festen Alkohols mit einem Schmelzpunkt im Bereich von 45 °C bis 65 °C, bis zu 5 Gew.-%, insbesondere 1 Gew.-% bis 3 Gew.-% Emulgator für den Alkohol, bis zu 5 Gew.-%, insbesondere 0,2 Gew.-% bis 3 Gew.-% Dispergiermittel für den Silberkorrosionsinhibitor beziehungsweise das anorganische Pigment und bis zu 7 Gew.-% Wasser enthält.

Der enzymhaltige Kern kann unter Einsatz beliebiger, mit dem Enzym verträglicher Trägermaterialien auf jede bekannte Weise hergestellt werden. Bevorzugt wird er zumindest anteilsweise aus Gründen der höheren Enzymstabilität gemäß den einen Extrusionsschritt umfassenden Verfahren der internationalen Patentanmeldung WO 92/11347 beziehungsweise der deutschen Patentanmeldung DE 43 10 506.8 = WO-A-94/23005 unter Einsatz quellfähiger Stärke, wasserlöslichem organischem Polymer und Getreidemehl hergestellt.

Die Erfindung betrifft somit weiterhin ein Verfahren zur Herstellung eines für die Einarbeitung in partikelförmige Reinigungsmittel geeigneten Enzymgranulates mit einer mittleren Korngröße von 0,8 mm bis 1,2 mm durch Extrudieren eines durch Vermischen einer gegebenenfalls zuvor durch Mikrofiltration von unlöslichen Bestandteilen befreiten, aufkonzentrierten Fermentationsbrühe mit anorganischem und/oder organischem Trägermaterial als Zuschlagstoff entstandenen Enzym-Vorgemischs, gegebenenfalls Sphäronisierung des Extrudats in einem Rondiergerät, Trocknung und Aufbringen einer äußeren Umhüllungsschicht, wobei man in einer Wirbelschicht aus Extrudat eine äußere Umhüllungsschicht eines Überzugssystems aus mindestens 50 Gew.-% Silberkorrosionsinhibitor, bis zu 25 Gew.-%, insbesondere 5 Gew.-% bis 20 Gew.-% feinteiligem anorganischem Pigment, 5 Gew.-% bis 20 Gew.-% bei Raumtemperatur festem Alkohol mit einem Schmelzpunkt im Bereich von 45 °C bis 65 °C, bis zu 5 Gew.-%, insbesondere 1 Gew.-% bis 3 Gew.-% Emulgator für den Alkohol, bis zu 5 Gew.-%, insbesondere 0,2 Gew.-% bis 3 Gew.-% Dispergiermittel für den Silberkorrosionsinhibitor beziehungsweise das anorganische Pigment und bis zu 7 Gew.-% Wasser aufbringt. In einer bevorzugten Ausgestaltung dieses Verfahrens geht man so vor, daß man einen durch Extrusion hergestellten Enzymkern mit einem teilchenförmig konfektioniertem zweiten Enzym und gegebenenfalls weiteren teilchenförmig konfektionierten Enzymen unter Agglomerationsbedingungen vermischt, wobei gegebenenfalls ein Bindemittel zum Einsatz kommt, und den Silberkorrosionsinhibitor enthaltenden Überzug anschließend oder gleichzeitig aufbringt, wobei die mittlere Teilchengröße des das erste Enzym enthaltenden Extrudatkerns vorzugsweise das 1,1- bis 3-fache, insbesondere das 1,3- bis 2-fache derjenigen des zweiten oder weiteren teilchenförmig konfektionierten Enzyms beträgt. Diese Variante stützt sich auf das in der deutschen Patentanmeldung DE 43 29 463.4 = WO-A-95/06709 offenbarte Herstellungsverfahren für Mehrenzymgranulate. Vorzugsweise handelt es sich bei dem Enzym im Extrudatkern um Protease und bei dem in den separat hergestellten, kleineren Teilchen, welche an das Extrudat agglomerieren, enthaltenen Enzym beziehungsweise bei den Enzymen, falls mehrere verschiedene kleinere Teilchen eingesetzt werden, um Amylase, Lipase, Cellulase und/oder Oxidase.

Die Alkoholkomponente des Überzugssystems ist vorzugsweise ein primärer linearer Alkohol mit 14 bis 22 C-Atomen oder ein Gemisch aus diesen. Zu den genannten Alkoholen gehören insbesondere Myristylakohol, Cetylalkohol, Stearylalkohol, Arachidylalkohol, Behenylalkohol und ein- bis dreifach ungesättigte Alkohole entsprechender Kettenlänge, wobei wesentlich ist, daß die genannte Alkoholkomponente des Überzugssystems einen Schmelzpunkt im Bereich von 45 °C bis 65 °C, insbesondere von 50 °C bis 60 °C aufweist, worunter hier die Temperatur verstanden werden soll, bei der beim Erwärmen 100 % der Alkoholkomponente in flüssiger Form vorliegen. Bei Einsatz von Alkoholgemischen sind auch solche brauchbar, welche geringe Anteile, normalerweise unter 15 Gew.-% bezogen auf Alkoholgemisch, an bei Raumtemperatur flüssigen Anteilen enthalten, solange das gesamte Alkoholgemisch bei Raumtemperatur fest erscheint und einen Erstarrungspunkt im Bereich von 45 °C bis 65 °C, insbesondere von 50 °C bis 60 °C aufweist. Der Erstarrungspunkt ist die Temperatur, bei der beim Abkühlen auf eine Temperatur oberhalb des Schmelzpunkts erwämten Materials eine Verfestigung eintritt. Er kann mit Hilfe eines rotierenden Thermometers nach dem Verfahren der DIN ISO 2207 bestimmt werden.

Geeignete Emulgatoren für die Alkoholkomponente sind Substanzen, welche in der Lage sind, die Alkoholkomponente in Wasser zu emulgieren, so daß eine bei Temperaturen bis zu 95 °C versprühbare Mischung entsteht, und/oder welche es erlauben, das Umhüllungssystem in eine möglichst homogene, bei Temperaturen bis zu 120 °C versprühbare Schmelze zu überführen. Als Anhaltspunkt kann in diesem Zusammenhang dienen, daß Flüssigkeiten mit Viskositäten bis zu etwa 10 000 cPs bei den genannten Temperaturen in der Regel problemlos mittels dafür vorgesehener Vorrichtungen versprüht und auf Enzymgranulate aufgebracht werden können. Zu den Emulgatoren für die Alkoholkomponente des Überzugssystems gehören beispielsweise die Ethoxylierungsprodukte der genannten Alkohole, wobei deren Umsetzungsprodukte mit durchschnittlich 25 bis 80, insbesondere 30 bis 45 Molequivalenten Ethylenoxid bevorzugt sind. Falls das Umhüllungssystem als wäßrige Dispersion auf das Enzymgranulat aufgebracht wird, sind unter den genannten Verbindungen solche mit Ethoxylierungsgraden von 25 bis 50, das heißt Umsetzungsprodukte mit 25 bis 50 Molequivalenten Ethylenoxid, bevorzugt. Alternativ oder zusätzlich zu den Alkoholethoxylaten können auch ethoxylierte Fettsäuren, wobei der Ethoxylierungsgrad vorzugsweise 3 bis 9 beträgt, ethoxylierte Fettsäureamide, wobei der Ethoxylierungsgrad vorzugsweise 4 bis 11 beträgt, und/oder Ethoxylierungsprodukte von Hydroxyfettsäureestern mit 1 bis 6 C-Atomen im Alkoholteil des Esters, beispielsweise Ricinolsäureglycerid, wobei der Ethoxylierungsgrad vorzugsweise 5 bis 80 insbesondere 20 bis 40 beträgt, als Emulgatorkomponente im Überzugssystem eingesetzt werden. Die Fettsäurekomponente der genannten Substanzen besitzt vorzugsweise 12 bis 22 C-Atome. Gewünschtenfalls können die Ethoxygruppen in den genannten Emulgatoren zumindest teilweise durch Propoxygruppen ersetzt sein.

Zu den anorganischen Pigmenten, mit denen eventuelle störende Färbungen des Enzymgranulats überdeckt werden können, gehören beispielsweise Calciumcarbonat, Titandioxid, welches in Rutil- oder Anatase-Kristallmodifikation vorliegen kann, Zinkoxid, Zinksulfid, Bleiweiß (basisches Bleicarbonat), Bariumsulfat, Aluminiumhydroxid, Antimonoxid, Lithopone (Zinksulfid-Bariumsulfat), Kaolin, Kreide und/oder Glimmer. Diese liegen in so feinteiliger Form vor, daß sie in einer Schmelze der organischen Bestandteile des Überzugssystems oder in Wasser dispergiert werden können. Üblicherweise liegt die mittlere Teilchengröße derartiger Pigmente im Bereich von 0,004 µm bis 50 µm. Insbesondere wenn das Pigment beziehungsweise das gesamte Überzugssystem in Form einer wäßrigen Dispersion eingesetzt werden soll, ist es bevorzugt, daß diese Dispersion Dispergiermittel für das Pigment und/oder den Silberkorrosionsinhibitor enthält. Derartige Dispergiermittel können anorganisch, beispielsweise Aluminiumoxid oder Siliziumoxid, welche auch als Pigmente dienen können, oder organisch, beispielsweise Diethylenglykol oder Dipropylenglykol, sein. Der Einsatz mit Dispergiermitteln oberflächenmodifizierter Pigmente ist ebenfalls möglich. Vorzugsweise wird mit Al-, Si-, Zr- oder Polyol-Verbindungen oberflächenmodifiziertes Titandioxidpigment, insbesondere in Rutilform, wie es beispielsweise unter den Handelsnamen Kronos^{(R)} 2132 (Fa. Kronos-Titan) oder Hombitan^{(R)} R 522 (Sachtleben Chemie GmbH) vertrieben wird, eingesetzt. Brauchbar sind auch die Tiona^{(R)} RLL-, AG- bzw. VC-Typen der Fa. Solvay sowie die Bayertitan^{(R)} RD-, R-KB- und AZ-Typen der Fa. Bayer AG.

Als Enzyme kommen in erster Linie die aus Mikroorganismen, wie Bakterien oder Pilzen, gewonnenen Proteasen, Lipasen, Amylasen und/oder Cellulasen in Frage, wobei von Bacillus-Arten erzeugte Proteasen sowie ihre Gemische mit Amylasen bevorzugt sind. Sie werden in bekannter Weise durch Fermentationsprozesse aus geeigneten Mikroorganismen gewonnen, die zum Beispiel in den deutschen Offenlegungsschriften DE 19 40 488, DE 20 44 161, DE 22 01 803 und DE 21 21 397, den US-amerikanischen Patentschriften US 3 632 957 und US 4 264 738 sowie der europäischen Patentanmeldung EP 006 638 beschrieben sind. Besonders vorteilhaft kann das erfindungsgemäße Verfahren zur Formulierung von sehr aktiven Proteasen, die beispielsweise aus der internationalen Patentanmeldung WO 91/2792 bekannt sind, angewendet werden, weil deren lagerstabile Einarbeitung in Wasch- und Reinigungsmittel oft Probleme bereitet und erfindungsgemäß die Entstehung unerwünschter Enzymstäube vermieden wird. Enzyme sind in den erfindungsgemäßen Granulaten vorzugsweise in Mengen von 4 Gew.-% bis 20 Gew.-% enthalten. Falls es sich bei dem erfindungsgemäßen Enzymgranulat um eine proteasehaltige Formulierung handelt, beträgt die Proteaseaktivität vorzugsweise 150 000 Proteaseeinheiten (PE, bestimmt nach der in Tenside 7 (1970), 125 beschriebenen Methode) bis 350 000 PE, insbesondere 160 000 PE bis 300 000 PE, pro Gramm Enzymgranulat.

Als Trägermaterialien für das Enzym sind im Prinzip alle organischen oder anorganischen pulverförmigen Substanzen brauchbar, welche die zu granulierenden Enzyme nicht oder nur tolerierbar wenig zerstören oder desaktivieren und unter Granulationsbedingungen stabil sind. Zu derartigen Substanzen gehören beispielsweise Stärke, Getreidemehl, Cellulosepulver, Alkalialumosilikat, insbesondere Zeolith, Schichtsilikat, zum Beispiel Bentonit oder Smectit, und wasserlösliche anorganische oder organische Salze, zum Beispiel Alkalichlorid, Alkalisulfat, Alkalicarbonat oder Alkaliacetat, wobei Natrium oder Kalium die bevorzugten Alkalimetalle sind. Bevorzugt wird ein Trägermaterialgemisch aus in Wasser quellfähiger Stärke, Getreidemehl und gegebenenfalls Cellulosepulver sowie Alkalicarbonat eingesetzt.

Bei der in Wasser quellfähigen Stärke handelt es sich vorzugsweise um Maisstärke, Reisstärke, Kartoffelstärke oder Gemische aus diesen, wobei der Einsatz von Maisstärke besonders bevorzugt ist. Quellfähige Stärke ist in den erfindungsgemäßen Enzymgranulaten vorzugsweise in Mengen von 20 Gew.-% bis 50 Gew.-%, insbesondere von 25 Gew.-% bis 45 Gew.-% enthalten. Dabei beträgt die Summe der Mengen der quellfähigen Stärke und des Mehls vorzugsweise nicht über 80 Gew.-%, insbesondere 32 Gew.-% bis 65 Gew.-%.

Bei dem Getreidemehl handelt es sich insbesondere um ein aus Weizen, Roggen, Gerste oder Hafer herstellbares Produkt oder um ein Gemisch dieser Mehle, wobei Vollkornmehle bevorzugt sind. Unter einem Vollkornmehl wird dabei ein nicht voll ausgemahlenes Mehl verstanden, das aus ganzen, ungeschälten Körnern hergestellt worden ist oder zumindest überwiegend aus einem derartigen Produkt besteht, wobei der Rest aus voll ausgemahlenem Mehl beziehungsweise Stärke besteht. Vorzugsweise werden handelsübliche Weizenmehl-Qualitäten, wie Type 450 oder Type 550, eingesetzt. Auch die Verwendung von Mehlprodukten der zu vorgenannten quellfähigen Stärken führenden Getreidearten ist möglich, wenn darauf geachtet wird, daß die Mehle aus den ganzen Körnern hergestellt worden sind. Durch die Mehlkomponente des Zuschlagstoffgemisches wird bekanntermaßen eine wesentliche Geruchsreduzierung der Enzymzubereitung erreicht, welche die Geruchsverminderung durch die Einarbeitung gleicher Mengen entsprechender Stärkearten bei weitem übertrifft. Derartiges Getreidemehl ist in den erfindungsgemäßen Enzymgranulaten vorzugsweise in Mengen von 10 Gew.-% bis 35 Gew.-%, insbesondere von 15 Gew.-% bis 25 Gew.-% enthalten.

Die erfindungsgemäßen Enzymgranulate enthalten als weitere Komponente des Trägermaterials vorzugsweise 1 Gew.-% bis 50 Gew.-%, vorzugsweise 5 Gew.-% bis 25 Gew.-%, bezogen auf gesamtes Granulat, eines Granulierhilfsmittelsystems, das Alkali-Carboxymethylcellulose mit Substitutionsgraden von 0,5 bis 1 und Polyethylenglykol und/oder Alkylpolyethoxylat enthält. In diesem Granulierhilfsmittelsystem sind vorzugsweise, jeweils bezogen auf fertiges Enzymgranulat, 0,5 Gew.-% bis 5 Gew.-% Alkali-Carboxymethylcellulose mit Substitutionsgraden von 0,5 bis 1 und bis zu 3 Gew.-% Polyethylenglykol und/oder Alkylpolyethoxylat enthalten, wobei besonders bevorzugt ist, wenn mindestens 0,5 Gew.-%, insbesondere 0,8 Gew.-% bis 2 Gew.-% Polyethylenglykol mit einer mittleren Molmasse unter 1000 und/oder Alkylpolyethoxylat mit mindestens 30 Ethoxygruppen vorhanden ist, falls mehr als 2 Gew.-% Alkali-Carboxymethylcellulose enthalten ist.

Gegebenenfalls können als zusätzliche Bestandteile des Granulierhilfsmittel systems auch weitere Cellulose- oder Stärkeether, wie Carboxymethylstärke, Methylcellulose, Hydroxyethylcellulose, Hydroxypropylcellulose sowie entsprechende Cellulosemischether, Gelatine, Casein, Traganth, Maltodextrose, Saccharose, Invertzucker, Glukosesirup oder andere in Wasser lösliche beziehungsweise gut dispergierbare Oligomere oder Polymere natürlichen oder synthetischen Ursprungs verwendet werden. Brauchbare synthetische wasserlösliche Polymere sind Polyacrylate, Polymethacrylate, Copolymere der Acrylsäure mit Maleinsäure oder vinylgruppenhaltige Verbindungen, ferner Polyvinylalkohol, teilverseiftes Polyvinylacetat und Polyvinylpyrrolidon. Soweit es sich bei den vorgenannten Verbindungen um solche mit freien Carboxylgruppen handelt, liegen sie normalerweise in Form ihrer Alkalisalze, insbesondere ihrer Natriumsalze vor. Derartige zusätzliche Granulierhilfsmittel können in den erfindungsgemäßen Enzymgranulaten in Mengen bis zu 10 Gew.-%, insbesondere von 0,5 Gew.-% bis 8 Gew.-% enthalten sein. Höhermolekulare Polyethylenglykole, das heißt solche mit einem mittleren Molekulargewicht über 1000, sind zwar als synthetische wasserlösliche Polymere mit staubbindender Wirkung brauchbar, doch bewirken gerade die höhermolekularen Polyethylenglykole eine unerwünschte Erhöhung der benötigten Granulatauflösezeit, so daß diese Substanzen in den erfindungsgemäßen Enzymgranulaten vorzugsweise völlig fehlen.

Zur Herstellung der erfindungsgemäßen Enzymgranulate geht man vorzugsweise von Fermentbrühen aus, die beispielsweise durch Mikrofiltration von unlöslichen Begleitstoffen befreit werden. Die Mikrofiltration wird dabei vorzugsweise als Querstrom-Mikrofiltration unter Verwendung poröser Rohre mit Mikroporen größer 0,1 µm, Fließgeschwindigkeiten der Konzentratlösung von mehr als 2 m/s und einem Druckunterschied zur Permeatseite von unter 5 bar durchgeführt, wie beispielsweise in der europäischen Patentanmeldung EP 200 032 beschrieben. Anschließend wird das Mikrofiltrationspermeat vorzugsweise durch Ultrafiltration, gegebenenfalls mit anschließender Vakuumeindampfung, aufkonzentriert. Die Aufkonzentration kann dabei, wie in der internationalen Patentanmeldung WO 92/11347 beschrieben, so geführt werden, daß man nur zu relativ niedrigen Gehalten an Trockensubstanz von vorzugsweise 5 Gew.-% bis 50 Gew.-%, insbesondere von 10 Gew.-% bis 40 Gew.-% gelangt. Das Konzentrat wird einem zweckmäßigerweise zuvor hergestellten trockenen, pulverförmigen bis körnigen Gemisch der oben beschriebenen Zuschlagstoffe zudosiert. Der Wassergehalt der Mischung sollte so gewählt werden, daß sie sich bei der Bearbeitung mit Rühr- und Schlagwerkzeugen in körnige, bei Raumtemperatur nicht klebende Partikel überführen und bei Anwendung höherer Drücke plastisch verformen und extrudieren läßt. Das rieselfähige Vorgemisch wird im Prinzip bekannter Weise anschließend in einem Kneter sowie einem angeschlossenen Extruder zu einer plastischen, möglichst homogenen Masse verarbeitet, wobei als Folge der mechanischen Bearbeitung sich die Masse auf Temperaturen zwischen 40°C und 60°C, insbesondere 45°C bis 55°C erwärmen kann. Das den Extruder verlassende Gut wird durch eine Lochscheibe mit nachfolgendem Abschlagmesser geführt und dadurch zu zylinderförmigen Partikeln definierter Größe zerkleinert. Zweckmäßigerweise beträgt der Durchmesser der Bohrungen in der Lochscheibe 0,7 mm bis 1,2 mm, vorzugsweise 0,8 mm bis 1,0 mm. Die in dieser Form vorliegenden Partikel können anschließend getrocknet und mit dem Überzugssystem gemäß der Erfindung umhüllt werden. Es hat sich jedoch als vorteilhaft erwiesen, die den Extruder und Zerhacker verlassenden zylindrischen Partikel vor dem Umhüllen zu sphäronisieren, das heißt sie in geeigneten Vorrichtungen abzurunden und zu entgraten. Man verwendet hierzu eine Vorrichtung, die aus einem zylindrischen Behälter mit stationären, festen Seitenwänden und einer bodenseitig drehbar gelagerten Reibplatte bestehen. Vorrichtungen dieser Art sind unter der Warenbezeichnung Marumerizer^{(R)} in der Technik verbreitet und beispielsweise in den deutschen Auslegeschriften DE 21 37 042 und DE 21 37 043 beschrieben. Anschließend können eventuell auftretende staubförmige Anteile mit einer Korngröße unter 0,1 mm, insbesondere unter 0,4 mm sowie eventuelle Grobanteile mit einer Korngröße über 2 mm, insbesondere über 1,6 mm durch Sieben oder Windsichten entfernt und gegebenenfalls in den Herstellungsprozess zurückgeführt werden. Nach der Sphäronisierung werden die Kügelchen kontinuierlich oder chargenweise, vorzugsweise unter Verwendung einer Wirbelschichttrockenanlage, bei Zulufttemperaturen von vorzugsweise 35 °C bis 50 °C und insbesondere bei einer Produkttemperatur von nicht über 42 °C bis zum gewünschten Restfeuchtegehalt von beispielsweise 4 Gew.-% bis 10 Gew.-%, insbesondere 5 Gew.-% bis 8 Gew.-%, bezogen auf gesamtes Granulat, getrocknet.

Nach oder vorzugsweise während der Trocknung wird das den Silberkorrosionsinhibitor enthaltende Überzugssystem als äußere Umhüllung aufgebracht. In einer Ausgestaltung des erfindungsgemäßen Herstellungsverfahrens wird das Überzugssystem als wäßrige Lösung beziehungsweise Dispersion, die vorzugsweise 30 Gew.-% bis 65 Gew.-% Wasser und 35 Gew.-% bis 70 Gew.-% des Überzugssystems enthält, wobei im Überzugssystem insbesondere 1 Gew.-% bis 2,5 Gew.-%, bezogen auf gesamtes Überzugssystem, Dispergiermittel für den Silberkorrosionsinhibitor und/oder das Pigment enthalten ist, in die Wirbelschicht aus enzymhaltigem Kern eingebracht. Das über die wäßrige Dispersion zugeführte Wasser wird bei der gleichzeitig vorgenommenen oder einer anschließend erneut erforderlichen Trocknung wieder entfernt. In einer weiteren Ausgestaltung des erfindungsgemäßen Herstellungsverfahrens wird das Überzugssystem, gegebenenfalls unter Kühlung, als erwärmte, bei einer Temperatur von 5 °C bis 45 °C über dem Schmelzpunkt der Alkoholkomponente vorliegende Flüssigkeit auf den enzymhaltigen Kern aufgebracht. Außerdem ist eine Kombination dieser Vorgehensweisen, die darin besteht, einen Teil des Überzugssystems, der dann vorzugsweise allein aus dem Silberkorrosionsinhibitor besteht, in Form einer wäßrigen Lösung oder Dispersion und einen zweiten Teil, der dann vorzugsweise die übrigen Komponenten des Überzugssystems umfaßt, in Form einer Schmelze aufzubringen, möglich. Vorzugsweise bringt man, bezogen auf fertiges Granulat, 10 Gew.-% bis 50 Gew.-% des Überzugssystems als äußere Umhüllungsschicht auf das enzymhaltige Extrudat auf.

Die nach dem erfindungsgemäßen Verfahren erhaltene Enzymzubereitung besteht aus weitgehend abgerundeten, gleichmäßig umhüllten und staubfreien Partikeln, die in der Regel ein Schüttgewicht von etwa 500 bis 900 Gramm pro Liter, insbesondere 650 bis 880 Gramm pro Liter aufweisen. Die erfindungsgemäßen Granulate zeichnen sich durch eine sehr hohe Lagerstabilität, insbesondere bei Temperaturen über Raumtemperatur und hoher Luftfeuchtigkeit, sowie ein rasches Lösungsverhalten in der Reinigungsmittelflotte aus. Beispielsweise ist es möglich, erfindungsgemäße Granulate herzustellen, die 100 % ihrer Enzymaktivität innerhalb von 3 Minuten, insbesondere innerhalb von 90 Sekunden bis 2 Minuten, in Wasser bei 25 °C freisetzen.

Das erfindungsgemäße oder nach dem erfindungsgemäßen Verfahren hergestellte Enzymgranulat wird vorzugsweise zur Herstellung fester, insbesondere teilchenförmiger Reinigungsmittel verwendet, die durch einfaches Vermischen der Enzymgranulate mit in derartigen Mitteln üblichen weiteren Pulverkomponenten erhalten werden können. Für die Einarbeitung in teilchenförmige Mittel weist das Enzymgranulat vorzugsweise mittlere Korngrößen im Bereich von 0,8 mm bis 1,6 mm auf. Die erfindungsgemäßen Granulate enthalten vorzugsweise weniger als 2 Gew.-%, insbesondere höchstens 1,4 Gew.-% an Partikeln mit Korngrößen außerhalb des Bereichs von 0,4 mm bis 1,6 mm.

Da sich die Silberkorrosionsinhibitoren wie oben ausgführt insbesondere zur Verhinderung der Silberkorrosion eignen, wenn sie in niederalkalischen Reinigern zum maschinellen Reinigen von Geschirr enthalten sind, ist ein weiterer Erfindungsgegenstand ein niederalkalisches Mittel zum maschinellen Reinigen von Geschirr, deren 1-gewichtsprozentige Lösung einen pH-Wert von 8 bis 11,5, vorzugsweise 9 bis 10,5 aufweist, enthaltend Enzym, Silberkorrosionsinhibitor, 15 Gew.-% bis 60 Gew.-%, vorzugsweise 30 Gew.-% bis 50 Gew.-% wasserlösliche Builderkomponente, 5 Gew.-% bis 25 Gew.-%, vorzugsweise 10 Gew.-% bis 15 Gew.-% Bleichmittel auf Sauerstoffbasis, 1 Gew.-% bis 10 Gew.-%, vorzugsweise 2 Gew.-% bis 6 Gew.-% Bleichaktivator, jeweils bezogen auf das gesamte Mittel, welches dadurch gekennzeichnet ist, daß es 0,1 Gew.-% bis 10 Gew.-%, insbesondere 0,5 Gew.-% bis 5 Gew.-% Enzymgranulat enthält, welches im Kern Enzym und anorganisches und/oder organisches Trägermaterial sowie gegebenenfalls Granulierhilfsmittel und eine gleichmäßige Umhüllungsschicht aufweist, die einen insbesondere anorganischen Silberkorrosionsinhibitor enthält. Auch dabei sind Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen, bevorzugt.

Als wasserlösliche Builderkomponenten in derartigen niederalkalischen Reinigungsmitteln kommen prinzipiell alle in maschinellen Geschirreinigungsmitteln üblicherweise eingesetzten Builder in Frage, zum Beispiel polymere Alkaliphosphate, die in Form ihrer alkalischen neutralen oder sauren Natrium- oder Kaliumsalze vorliegen können. Beispiele hierfür sind Tetranatriumdiphosphat, Dinatriumdihydrogendiphosphat, Pentanatriumtriphosphat, sogenanntes Natriumhexametaphosphat sowie die entsprechenden Kaliumsalze beziehungsweise Gemische aus Natrium- und Kaliumsalzen. Ihre Mengen können im Bereich von bis zu etwa 35 Gew.-%, bezogen auf das gesamte Mittel liegen; vorzugsweise sind die erfindungsgemäßen Mittel jedoch frei von solchen Phosphaten. Weitere mögliche wasserlösliche Builderkomponenten sind zum Beispiel organische Polymere nativen oder synthetischen Ursprungs, vor allem Polycarboxylate, die insbesondere in Hartwasserregionen als Co-Builder wirken. In Betracht kommen beispielsweise Polyacrylsäuren und Copolymere aus Maleinsäureanhydrid und Acrylsäure sowie die Natriumsalze dieser Polymersäuren. Handelsübliche Produkte sind zum Beispiel Sokalan^{(R)} CP 5 und PA 30 der Firma BASF. Zu den als Co-Builder brauchbaren Polymeren nativen Ursprungs gehören beispielsweise oxidierte Stärke, beispielsweise gemäß der deutschen Patentanmeldung P 42 28 786, und Polyaminosäuren wie Polyglutaminsäure oder Polyasparaginsäure. Weitere mögliche Builderkomponenten sind natürlich vorkommende Hydroxycarbonsäuren wie zum Beispiel Mono-, Dihydroxybernsteinsäure, α-Hydroxypropionsäure und Gluconsäure. Zu den bevorzugten Builderkomponenten gehören die Salze der Citronensäure, insbesondere Natriumcitrat. Als Natriumcitrat kommen wasserfreies Trinatriumcitrat beziehungsweise vorzugsweise Trinatriumcitratdihydrat in Betracht. Trinatriumcitratdihydrat kann als fein- oder grobkristallines Pulver eingesetzt werden. In Abhängigkeit vom letztlich in den erfindungsgemäßen Mitteln eingestellten pH-Wert können auch die zu den genannten Co-Builder-Salzen korrespondierenden Säuren vorliegen.

Als Bleichmittel auf Sauerstoffbasis kommen in erster Linie Natriumperboratmono- beziehungsweise -tetrahydrat und/oder Natriumpercarbonat in Betracht. Der Einsatz von Natriumpercarbonat hat Vorteile, da sich dieses besonders günstig auf das Korrosionsverhalten an Gläsern auswirkt. Das Bleichmittel auf Sauerstoffbasis ist deshalb vorzugsweise ein Percarbonat-Salz, insbesondere Natriumpercarbonat. Da Bleichmittel auf Sauerstoffbasis in der Regel erst bei erhöhten Temperaturen ihre volle Wirkung entfalten, werden zu ihrer Aktivierung bei ca. 60°C, das heißt der ungefähren Temperaturen des Reinigungsprozesses in der Geschirrspülmaschine, sogenannte Bleichaktivatoren eingesetzt. Als Bleichaktivatoren können zum Beispiel Pentaacetylglucose, 1,5-Diacetyl-2,2-dioxo-hexahydro-1,3,5-triazin (DADHT) und/oder Isatosäureanhydrid dienen, vorzugsweise jedoch N,N,N',N'-Tetraacetylethylendiamin (TAED). Überdies kann auch der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat zweckdienlich sein.

Vorzugsweise enthalten die erfindungsgemäßen Mittel die in üblichen niederalkalischen maschinellen Geschirreinigungsmitteln enthaltenen Alkaliträger wie zum Beispiel Alkalisilikate, Alkalicarbonate und/oder Alkalihydrogencarbonate. Zu den üblicherweise eingesetzten Alkaliträgern zählen Carbonate, Hydrogencarbonate und Alkalisilikate mit einem Molverhältnis SiO₂ / M₂O (M = Alkaliatom) von 1,5 : 1 bis 2,5 : 1. Alkalisilikate können dabei in Mengen von bis zu 30 Gew.-%, bezogen auf das gesamte Mittel, enthalten sein. Auf den Einsatz der hoch alkalischen Metasilikate als Alkaliträger wird vorzugsweise ganz verzichtet. Das in den erfindungsgemäßen Mitteln bevorzugt eingesetzte Alkaliträgersystem ist ein Gemisch aus Carbonat und Hydrogencarbonat, vorzugsweise Natriumcarbonat und Hydrogencarbonat, das in einer Menge von bis zu 60 Gew.-%, vorzugsweise 10 Gew.-% bis 40 Gew.-%, enthalten ist. Je nachdem, welcher pH-Wert letztendlich gewünscht wird, variiert das Verhältnis von eingesetztem Carbonat und eingesetztem Hydrogencarbonat, üblicherweise wird jedoch ein Überschuß an Natriumhydrogencarbonat eingesetzt, so daß das Gewichtsverhältnis zwischen Hydrogencarbonat und Carbonat im allgemeinen 1 : 1 bis 15 : 1 beträgt.

Zusätzlich können die erfindungsgemäßen Mittel in fester Form konfektionierte Enzyme wie Proteasen, Amylasen, Lipasen und Cellulasen enthalten, die nicht in Form des erfindungsgemäßen Granulates vorliegen, beispielsweise Proteasen wie BLAP^{(R)}, Optimase^{(R)}, Opticlean^{(R)}, Maxacal^{(R)}, Maxapem^{(R)} Esperase^{(R)} und/oder Savinase^{(R)}, Amylasen wie Termamyl^{(R)}, Amylase-LT^{(R)} und/oder Maxamyl^{(R)}, Lipasen wie Lipolase^{(R)} und/oder Lipozym^{(R)}, Cellulasen wie Celluzym^{(R)} und/oder KAC^{(R)}.

Den erfindungsgemäßen Mitteln können gegebenenfalls auch Tenside, insbesondere schwach schäumende nichtionische Tenside zugesetzt werden, die der besseren Ablösung fetthaltiger Speisereste, als Netzmittel und gegebenfalls im Rahmen der Herstellung der Reinigungsmittel als Granulierhilfsmittel dienen. Ihre Menge kann bis zu 5 Gew.-%, vorzugsweise bis zu 2 Gew.-% betragen. Üblicherweise werden extrem schaumarme Verbindungen eingesetzt. Hierzu zählen vorzugsweise C₁₂-C₁₈-Alkylpolyethylenglykol-polypropylenglykolether mit jeweils bei zu 8 Mol Ethylenoxid- und Propylenoxideinheiten im Molekül. Man kann aber auch andere als schaumarm bekannte nichtionische Tenside verwenden, wie zum Beispiel C₁₂-C₁₈-Alkylpolyethylenglykol-polybutylenglykolether mit jeweils bis zu 8 Mol Ethylenoxid- und Butylenoxideinheiten im Molekül, endgruppenverschlossene Alkylpolyalkylenglykolmischether sowie die zwar schäumenden, aber ökologisch attraktiven C₈-C₁₄-Alkylpolyglucoside mit einem Polymerisierungsgrad von etwa 1 bis 4 (z. B. APG^{(R)} 225 und APG^{(R)} 600 der Firma Henkel) und/oder C₁₂-C₁₄-Alkylpolyethylenglykole mit 3 - 8 Ethylenoxideinheiten im Molekül. Ebenfalls geeignet sind Tenside aus der Familie der Glucamide wie zum Beispiel Alkyl-N-Methyl-Glucamide, in denen der Alkylteil bevorzugt aus einem Fettalkohol mit der C-Kettenlänge C₆-C₁₄ stammt. Es ist teilweise vorteilhaft, wenn die beschriebenen Tenside als Gemische eingesetzt werden, zum Beispiel die Kombination Alkylpolyglykosid mit Fettalkoholethoxylaten oder Glucamid mit Alkylpolyglykosiden.

Sofern die Reinigungsmittel bei der Anwendung zu stark schäumen, können ihnen noch bis zu 6 Gew.-%, vorzugsweise etwa 0,5 Gew.-% bis 4 Gew.-% einer schaumdrückenden Verbindung, vorzugsweise aus der Gruppe der Silikonöle, Gemische aus Silikonöl und hydrophobierter Kieselsäure, Paraffine, Parafin-Alkohol-Kombinationen, hydrophobierter Kieselsäure, der Bisfettsäureamide, und sonstiger weiterer bekannter im Handel erhältliche Entschäumer zugesetzt werden. Weitere fakultative Inhaltsstoffe in den erfindungsgemäßen Mitteln sind z. B. Parfümöle.

Die erfindungsgemäßen Geschirrspülmittel liegen vorzugsweise als pulverförmige, granulare oder tablettenförmige Präparate vor, die in an sich bekannter Weise, beispielsweise durch Mischen, Granulieren, Walzenkompaktieren und/oder durch Sprühtrocknung der thermisch belastbaren Komponetent und Zumischen der empfindlicheren Komponenten, zu denen insbesondere das erfindungsgemäße Enzymgranulat zu rechnen ist, hergestellt werden können.

Zur Herstellung von erfindungsgemäßen Reinigungsmitteln in Tablettenform geht man vorzugsweise derart vor, daß man alle Bestandteile in einem Mischer miteinander vermischt und das Gemisch mittels herkömmlicher Tablet-tenpressen, beispielsweise Exzenterpressen oder Rundläuferpressen, mit Preßdrucken im Bereich von 200 · 10⁵ Pa bis 1 500 · 10⁵ Pa verpresst. Man erhält so problemlos bruchfeste und dennoch unter Anwendungsbedingungen ausreichend schnell lösliche Tabletten mit Biegefestigkeit von normalerweise über 150 N. Vorzugsweise weist eine derart hergestellte Tablette ein Gewicht von 15 g bis 40 g, insbesondere von 20 g bis 30 g auf, bei einem Durchmesser von 35 mm bis 40 mm.

Die Herstellung erfindungsgemäßer Maschinengeschirrspülmittel in Form von nicht staubenden, lagerstabil rieselfähigen Pulvern und/oder Granulaten mit hohen Schüttdichten im Bereich von 800 bis 1000 g/l kann dadurch erfolgen, daß man in einer ersten Verfahrensteilstufe die Builder-Komponenten mit wenigstens einem Anteil flüssiger Mischungskomponenten unter Erhöhung der Schüttdichte dieses Vorgemisches vermischt und nachfolgend - gewünschtenfalls nach einer Zwischentrocknung - die weiteren Bestandteile des Maschinengeschirrspülmittels, darunter die silberkorrosionsinhibitorhaltigen Enzymgranulate, mit dem so gewonnenen Vorgemisch vereinigt.

Die Mittel können sowohl in Haushaltsgeschirrspülmaschinen wie in gewerblichen Spülmaschinen eingesetzt werden. Die Zugabe erfolgt von Hand oder mittels geeigneten Dosiervorrichtungen. Die Anwendungskonzentrationen in der Reinigungsflotte betragen etwa 2 bis 8 g/l, vorzugsweise 2 bis 5 g/l.

Das Spülprogramm wird im allgemeinen durch einige auf den Reinigungsgang folgende Zwischenspülgänge mit klarem Wasser und einem Klarspülgang mit einem gebräuchlichem Klarspülmittel ergänzt und beendet. Nach dem Trocknen erhält man beim Einsatz erfindungsgemäßer Mittel nicht nur ein völlig sauberes und in hygienischer Hinsicht einwandfreies Geschirr, sondern vor allem auch hellglänzende Silberbesteckteile.

### Beispiele

### Beispiel 1

Ein nach der Fermentation gewonnener Erntebrei, wie in der internationalen Patentanmeldung-WO 91/2792 beschrieben, mit 75 000 Proteaseeinheiten pro g (PE/g) wurde nach der Entfernung der Fermentationsrückstände durch Dekantieren und Mikrofiltration in einer Ultrafiltrationsanlage aufkonzentriert. Nach der weiteren Aufkonzentrierung mittels Vakuumeindampfung enthielt die wäßrige Enzymsuspension 700 000 PE/g. Dieses Proteasekonzentrat wurde mit Zuschlagstoffen (3,5 Gew.-% Saccharose, 4,5 Gew.-% Cellulose, 3 Gew.-% Carboxymethylcellulose mit Substitutionsgrad 0,65-0,75, 19 Gew.-% Weizenmehl, 35 Gew.-% Maisstärke und 3 Gew.-% Polyethylenglykol, jeweils bezogen auf entstehendes Gemisch) vermischt, homogenisiert und anschließend in einem Extruder mit Schneidevorrichtung in Granulate überführt. Der Lochdurchmesser der Lochplatte des Extruders betrug 0,9 mm. Das Verhältnis von Länge zu Dicke des Granulatkorns lag bei 1. Nach der Verrundung und Trocknung der Granulate wurden die Partikel mit Teilchengrößen kleiner 0,4 mm und größer 1,6 mm abgesiebt. Die Kornfraktion zwischen 0,4 mm und 1,6 mm wurde in einem Wirbelschicht-Sprühgranulator des Typs STREA-1 der Fa. Aeromatic in der Wirbelschicht mit einer Lösung von Mangansulfat (240 g in 460 ml Wasser pro kg des Enzymkerns) gecoated. Während des Coatens wurden folgende Betriebsparameter eingestellt:

| | |
|---|---|
| Zulufttemperatur | 55-58 °C |
| Produkttemperatur | 36-40 °C |
| Ablufttemperatur | 32-36 °C |
| Luftmenge | 70 m³/h |
| Durchsatzrate an Coating-Lösung | 9,3 g/min |

Unter diesen Bedingungen verdampfte das Wasser der Mangansulfatlösung von der Granulatoberfläche und wurde mit der Abluft ausgetragen. Das Mangansulfat haftete fest am enzymhaltigen Kern.

Anschließend wurde in der gleichen Apparatur eine Coatingssuspension (285 g pro kg ursprünglichen Enzymkerns) aufgetragen, bestehend aus:

| | |
|---|---|
| Titandioxid | 17 % |
| technischem Stearylalkohol | 19 % |
| Eumulgin^{(R)} RT 40^{a)} | 3 % |
| Wasser | 61 % |

### a) 40-fach ethoxyliertes Ricinusöl, Hersteller Henkel KGaA

Der Stearylalkohol besaß die folgende C-Kettenverteilung: C₁₆ 0 - 5 %, C₁₈ 95 - 100 %, C₂₀ 0 - 2 %, eine Hydroxylzahl von 203 - 210 und einen Erstarrungsbereich von 55 - 57,5 °C.

Zur Herstellung dieser Coatingssuspension wurde das Wasser auf ca. 70 °C temperiert und mit dem flüssigen Emulgator vermischt. Der in fester Form vorliegende Stearylalkohol wurde in die Wasser/Emulgator-Lösung eingerührt und dabei aufgeschmolzen. Nach Zugabe des Titandioxids lag eine homogene Emulsion vor, in der das Titandioxidpigment gleichmäßig ohne Agglomeratbildung verteilt war. Diese Coatingsuspension wurde bei den oben angegebenen Betriebsparametern auf das mit Silberkorrosionsinhibitor umhüllte Enzymextrudat aufgesprüht. Das Wasser der Coatingsuspension verdampfte und wurde mit der Abluft ausgetragen. Nach Aufsprühen der Coatingsuspension waren die MnSO₄-modifizierten Enzymextrudate gleichmäßig mit einer weißen Farb- und Schutzschicht umhüllt. Der Fettalkohol bildete auf der Granulatoberfläche einen gleichmäßigen, unporösen Film aus.

### Beispiel 2:

Es wurde wie in Beispiel 1 gearbeitet. Vor Aufbringen des Mangansulfates aus wäßriger Lösung wurde das Proteaseextrudat mit einem Amylasegranulat (Maxamyl^{(R)} CXT 5000, Hersteller Gist Brocades, mittlere Teilchengröße 306 µm) im Gewichtsverhältnis 1,1 zu 1 vermischt und wie in Beispiel 1 zweistufig gecoated. Im Endprodukt lagen Amylase und Mangansulfat homogen auf der Proteaseextrudat-Oberfläche verteilt vor. Die zweite Coatingschicht schützt die Agglomerate vor dem Zerfall bei mechanischer Beanspruchung und erzeugt eine gleichmäßig weiße Farbgebung.

## Patentansprüche

1. Für die Einarbeitung in teilchenförmige Reinigungsmitte geeignetes Enzymgranulat, enthaltend im Kern Enzym und anorganisches und/oder organisches Trägermaterial sowie gegebenenfalls Granulierhilfsmittel, auf den eine gleichmäßige Umhüllungsschicht aufgebracht ist, dadurch gekennzeichnet, daß die Umhüllungsschicht einen Silberkorrosionsinhibitor enthält, welcher nicht CoSO₄ ist.

2. Enzymgranulat nach Anspruch 1, dadurch gekennzeichnet, daß der Silberkorrosionsinhibitor aus der Gruppe umfassend Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen, ausgewählt wird.

3. Enzymgranulat nach Anspruch 1 oder 2, dadurch gekennzeichnet, daß der Silberkorrosionsinhibitor MnSO₄, V₂O₅, V₂O₄, VO₂, TiOSO₄, K₂TiF₆, K₂ZrF₆, Co(NO₃)₂ und/oder Ce(NO₃)₃ ist.

4. Enzymgranulat nach einem der Ansprüche 1 bis 3, dadurch gekennzeichnet, daß es 40 Gew.-% bis 95 Gew.-%, insbesondere 50 Gew.-% bis 85 Gew.-% enzymhaltigen Kern und 5 Gew.-% bis 60 Gew.-%, insbesondere 15 Gew.-% bis 50 Gew.-% Silberkorrosionsinhibitor-haltige Umhüllungsschicht enthält.

5. Enzymgranulat nach einem der Ansprüche 1 bis 4, dadurch gekennzeichnet, daß die Umhüllungsschicht aus einem Umhüllungssystem besteht, welches 45 Gew.-% bis 90 Gew.-%, insbesondere 50 Gew.-% bis 80 Gew.-% Silberkorrosionsinhibitor, bis zu 25 Gew.-%, insbesondere 5 Gew.-% bis 20 Gew.-% feinteiliges anorganisches Pigment, 5 Gew.-% bis 20 Gew.-% eines bei Raumtemperatur festen Alkohols mit einem Schmelzpunkt im Bereich von 45 °C bis 65 °C, bis zu 5 Gew.-%, insbesondere 1 Gew.-% bis 3 Gew.-% Emulgator für den Alkohol, bis zu 5 Gew.-%, insbesondere 0,2 Gew.-% bis 3 Gew.-% Dispergiermittel für den Silberkorrosionsinhibitor beziehungsweise das anorganische Pigment und bis zu 7 Gew.-% Wasser enthält.

6. Enzymgranulat nach einem der Ansprüche 1 bis 5, dadurch gekennzeichnet, daß es Protease, Amylase, Lipase und/oder Cellulase enthält.

7. Enzymgranulat nach einem der Ansprüche 1 bis 6, dadurch gekennzeichnet, daß es Protease mit einer Aktivität von 150 000 PE bis 350 000 PE, insbesondere 160 000 PE bis 300 000 PE, pro Gramm Enzymgranulat enthält.

8. Verfahren zur Herstellung eines für die Einarbeitung in partikelförmige Reinigungsmittel geeigneten Enzymgranulates mit einer mittleren Korngröße von 0,8 mm bis 1,2 mm durch Extrudieren eines durch Vermischen einer gegebenenfalls zuvor durch Mikrofiltration von unlöslichen Bestandteilen befreiten, aufkonzentrierten Fermentationsbrühe mit anorganischem und/oder organischem Trägermaterial als Zuschlagstoff entstandenen Enzym-Vorgemischs, gegebenenfalls Sphäronisierung des Extrudats in einem Rondiergerät, Trocknung und Aufbringen einer äußeren Umhüllungsschicht, wobei man in einer Wirbelschicht aus Extrudat eine äußere Umhüllungsschicht eines Überzugssystems aus mindestens 50 Gew.-% Silberkorrosionsinhibitor, bis zu 25 Gew.-%, insbesondere 5'Gew.-% bis 20 Gew.-% feinteiligem anorganischem Pigment, 5 Gew.-% bis 20 Gew.-% bei Raumtemperatur festem Alkohol mit einem Schmelzpunkt im Bereich von 45 °C bis 65 °C, bis zu 5 Gew.-%, insbesondere 1 Gew.-% bis 3 Gew.-% Emulgator für den Alkohol, bis zu 5 Gew.-%, insbesondere 0,2 Gew.-% bis 3 Gew.-% Dispergiermittel für den Silberkorrosionsinhibitor beziehungsweise das anorganische Pigment und bis zu 7 Gew.-% Wasser aufbringt.

9. Verfahren nach Anspruch 8, dadurch gekennzeichnet, daß man einen durch Extrusion hergestellten Enzymkern mit einem teilchenförmig konfektioniertem zweiten Enzym und gegebenenfalls weiteren teilchenförmig konfektionierten Enzymen unter Agglomerationsbedingungen vermischt und den Silberkorrosionsinhibitor enthaltenden Überzug anschließend oder gleichzeitig aufbringt, wobei die mittlere Teilchengröße des das erste Enzym enthaltenden Extrudatkerns vorzugsweise das 1,1- bis 3-fache, insbesondere das 1,3- bis 2-fache derjenigen des zweiten oder weiteren teilchenförmig konfektionierten Enzyms beträgt.

10. Verfahren nach Anspruch 9, dadurch gekennzeichnet, daß das Enzym im Extrudatkern Protease und das in den separat hergestellten, kleineren Teilchen, welche an das Extrudat agglomerieren, enthaltene Enzym Amylase, Lipase, Cellulase und/oder Oxidase ist.

11. Verwendung eines Enzymgranulats gemäß einem der Ansprüche 1 bis 7 zur Herstellung fester, insbesondere teilchenförmiger Reinigungsmittel.

12. Niederalkalisches Mittel zum maschinellen Reinigen von Geschirr, enthaltend Enzym, Silberkorrosionsinhibitor, 15 Gew.-% bis 60 Gew.-%, vorzugsweise 30 Gew.-% bis 50 Gew.-% wasserlösliche Builderkomponente, 5 Gew.-% bis 25 Gew.-%, vorzugsweise 10 Gew.-% bis 15 Gew.-% Bleichmittel auf Sauerstoffbasis, 1 Gew.-% bis 10 Gew.-%, vorzugsweise 2 Gew.-% bis 6 Gew.-% Bleichaktivator, dadurch gekennzeichnet, daß es 0,1 Gew.-% bis 10 Gew.-%, insbesondere 0,5 Gew.-% bis 5 Gew.-% Enzymgranulat enthält, welches im Kern Enzym und anorganisches und/oder organisches Trägermaterial sowie gegebenenfalls Granulierhilfsmittel und eine gleichmäßige Umhüllungsschicht aufweist, die einen Silberkorrosionsinhibitor enthält.

## Claims

1. Enzyme granules suitable for incorporation in particulate detergents which, in their core, contain enzyme and inorganic and/or organic carrier material and optionally granulation auxiliaries, to which a uniform coating layer is applied, characterized in that the coating layer contains a silver corrosion inhibitor which is not CoSO₄.

2. Enzyme granules as claimed in claim 1, characterized in that the silver corrosion inhibitor is selected from the group consisting of manganese, titanium, zirconium, hafnium, vanadium, cobalt or cerium salts and/or complexes in which the metals mentioned are present in the one of the oxidation stages II, III, IV, V or VI.

3. Enzyme granules as claimed in claim 1 or 2, characterized in that the silver corrosion inhibitor is MnSO₄, V₂O₅, V₂O₄, VO₂, TiOSO₄, K₂TiF₆, K₂ZrF₆, Co(NO₃)₂ and/or Ce(NO₃)₃.

4. Enzyme granules as claimed in any of claims 1 to 3, characterized in that they contain 40% by weight to 95% by weight and, more particularly, 50% by weight to 85% by weight of enzyme-containing core and 5% by weight to 60% by weight and, more particularly, 15% by weight to 50% by weight of coating layer containing a silver corrosion inhibitor.

5. Enzyme granules as claimed in any of claims 1 to 4, characterized in that the coating layer consists of a coating system containing 45% by weight to 90% by weight and, more particularly, 50% by weight to 80% by weight of silver corrosion inhibitor, up to 25% by weight and, more particularly, 5% by weight to 20% by weight of fine-particle inorganic pigment, 5% by weight to 20% by weight of an alcohol solid at room temperature with a melting point in the range from 45°C to 65°C, up to 5% by weight and, more particularly, 1% by weight to 3% by weight of emulsifier for the alcohol, up to 5% by weight and, more particularly, 0.2% by weight to 3% by weight of dispersant for the silver corrosion inhibitor or the inorganic pigment and up to 7% by weight of water.

6. Enzyme granules as claimed in any of claims 1 to 5, characterized in that they contain protease, amylase, lipase and/or cellulase.

7. Enzyme granules as claimed in any of claims 1 to 6, characterized in that they contain protease with an activity of 150,000 PU to 350,000 PU and, more particularly, 160,000 PU to 300,000 PU per gram of enzyme granules.

8. A process for the production of enzyme granules with an average particle size of 0.8 mm to 1.2 mm suitable for incorporation in particulate detergents by extrusion of an enzyme compound prepared by mixing of a concentrated fermentation broth optionally freed beforehand from insoluble constituents by microfiltration with inorganic and/or organic carrier material as additive, optionally spheronization of the extrudate in a spheronizer, drying and application of an outer coating layer, an outer coating layer of a coating system of at least 50% by weight of silver corrosion inhibitor, up to 25% by weight and, more particularly, 5% by weight to 20% by weight of fine-particle inorganic pigment, 5% by weight to 20% by weight of alcohol solid at room temperature with a melting point of 45°C to 65°C, up to 5% by weight and, more particularly, 1% by weight to 3% by weight of emulsifier for the alcohol, up to 5% by weight and, more particularly, 0.2% by weight to 3% by weight of dispersant for the silver corrosion inhibitor or the inorganic pigment and up to 7% by weight of water being applied in a fluidized bed of extrudate.

9. A process as claimed in claim 8, characterized in that an enzyme core produced by extrusion is mixed with a particulate second enzyme and optionally other particulate enzymes under agglomeration conditions and the coating containing a silver corrosion inhibitor is subsequently or simultaneously applied, the average particle size of the extrudate core containing the first enzyme preferably being 1.1 to 3 times and, more preferably, 1.3 to 2 times the average particle size of the second or other particulate enzyme(s).

10. A process as claimed in claim 9, characterized in that the enzyme in the extrudate core is protease and the enzyme present in the separately produced, smaller particles which agglomerate onto the extrudate is amylase, lipase, cellulase and/or oxidase.

11. The use of the enzyme granules according to any of claims 1 to 7 for the production of solid detergents, more especially particulate detergents.

12. A low-alkali machine dishwashing detergent containing enzyme, silver corrosion inhibitor, from 15% by weight to 60% by weight and preferably from 30% by weight to 50% by weight of water-soluble builder component, from 5% by weight to 25% by weight and preferably from 10% by weight to 15% by weight of oxygen-based bleaching agent, from 1% by weight to 10% by weight and preferably from 2% by weight to 6% by weight of bleach activator, characterized in that it contains from 0.1% by weight to 10% by weight and, more particularly, from 0.5% by weight to 5% by weight of enzyme granules which, in their core, contain enzyme and inorganic and/or organic carrier material and optionally granulation auxiliaries and which have a uniform coating layer containing a silver corrosion inhibitor.

## Revendications

1. Granulé d'enzyme apte à l'incorporation dans des agents nettoyants granuleux, contenant dans son noyau une enzyme et une matière porteuse inorganique et/ou organique, ainsi que, le cas échéant, un adjuvant de granulation, sur lequel une couche d'enrobage uniforme est appliquée, qui est caractérisé en ce que la couche d'enrobage contient un inhibiteur de corrosion de l'argent, qui n'est pas du CoSO₄.

2. Granulé d'enzyme selon la revendication 1, caractérisé en ce que l'inhibiteur de corrosion de l'argent est sélectionné parmi le groupe comprenant les sels et/ou les complexes de manganèse, de titane, de zirconium, d'hafnium, de vanadium, de cobalt ou de cérium, dans lesquels les métaux cités sont présents dans l'un des stades d'oxydation II, III, IV, V ou VI.

3. Granulé d'enzyme selon la revendication 1 ou 2, caractérisé en ce que l'inhibiteur de corrosion de l'argent est le MnSO₄, le V₂O₅, le V₂O₄, le VO₂, le TiOSO₄, le K₂TiF₆, le K₂ZrF₆, le Co(NO₃)₂ et/ou le Ce(NO₃)₃.

4. Granulé d'enzyme selon une des revendications 1 à 3, caractérisé en ce qu'il renferme 40 à 95 % en poids, en particulier 50 à 85 % en poids de noyau renfermant l'enzyme et 5 à 60 % en poids, en particulier 15 à 50 % en poids de couche d'enrobage contenant l'inhibiteur de corrosion de l'argent.

5. Granulé d'enzyme selon une des revendications 1 à 4, caractérisé en ce que la couche d'enrobage est constituée d'un système d'enrobage qui renferme 45 à 90 % en poids, en particulier 50 à 80 % en poids d'inhibiteur de corrosion de l'argent, jusqu'à 25 % en poids, en particulier 5 à 20 % en poids de pigment inorganique à fines particules, 5 à 20 % en poids d'un alcool solide à température ambiante possédant, un point de fusion dans la plage de 45 à 65 °C, jusqu'à 5 % en poids, en particulier 1 à 3 % en poids d'émulsionnant pour l'alcool, jusqu'à 5 % en poids, en particulier 0,2 à 3 % en poids d'agent dispersant pour l'inhibiteur de corrosion de l'argent ou pour le pigment inorganique, et jusqu'à 7 % en poids d'eau.

6. Granulé d'enzyme selon une des revendications 1 à 5, caractérisé en ce qu'il renferme de la protéase, de l'amylase, de la lipase et/ou de la cellulase.

7. Granulé d'enzyme selon une des revendications 1 à 6, caractérisé en ce qu'il renferme de la protéase possédant une activité de 150 000 à 350 000 UP, en particulier de 160 000 à 300 000 UP par gramme de granulé d'enzyme.

8. Procédé de fabrication d'un granulé d'enzyme, apte à l'incorporation dans des agents nettoyants sous forme de particules, possédant une grosseur de grain moyenne de 0,8 à 1,2 mm, par extrusion d'un prémélange d'enzyme, obtenu par mélangeage d'un bouillon de fermentation concentré - le cas échéant préalablement débarrassé par microfiltration des constituants insolubles - avec une matière porteuse inorganique et/ou organique comme agrégat, le cas échéant, sphéronisation du produit d'extrusion dans un appareil à arrondir, séchage et application d'une couche d'enrobage externe, caractérisé en ce que l'on applique dans un lit fluidisé du produit d'extrusion une couche d'enrobage externe constituée d'un système de revêtement, qui renferme au moins 50 % en poids d'inhibiteur de corrosion de l'argent, jusqu'à 25 % en poids, en particulier 5 à 20 % en poids de pigment inorganique à fines particules, 5 à 20 % en poids d'alcool solide à température ambiante possédant un point de fusion dans la plage de 45 à 65 °C, jusqu'à 5 % en poids, en particulier 1 à 3 % en poids d'émulsionnant pour l'alcool, jusqu'à 5 % en poids, en particulier 0,2 à 3 % en poids d'agent dispersant pour l'inhibiteur de corrosion de l'argent ou pour le pigment inorganique, et jusqu'à 7 % en poids d'eau.

9. Procédé selon la revendication 8, caractérisé en ce que l'on mélange dans des conditions d'agglomération un noyau d'enzyme fabriqué par extrusion avec une deuxième enzyme formulée sous forme de particules et, le cas échéant, avec d'autres enzymes formulées sous forme de particules, et que l'on applique ensuite ou simultanément le revêtement renfermant l'inhibiteur de corrosion de l'argent, la grosseur de particule moyenne du noyau de produit d'extrusion renfermant la première enzyme atteignant de préférence 1,1 à 3 fois, en particulier 1,3 à 2 fois celle de la seconde ou des autres enzymes formulées sous forme de particules.

10. Procédé selon la revendication 9, caractérisé en ce que l'enzyme contenue dans le noyau du produit d'extrusion est la protéase et que celle contenue dans les particules plus petites fabriquées séparément, qui s'agglomèrent sur le produit d'extrusion, est l'amylase, la lipase, la cellulase et/ou l'oxydase.

11. Utilisation d'un granulé d'enzyme selon une des revendications 1 à 7 pour la fabrication d'agents de nettoyage solides, en particulier à fines particules.

12. Agent faiblement alcalin pour le nettoyage de la vaisselle en machine, contenant une enzyme, un inhibiteur de corrosion de l'argent, 15 à 60 % en poids, de préférence 30 à 50 % en poids de composant adjuvant soluble dans l'eau, 5 à 25 % en poids, de préférence 10 à 15 % en poids d'agent de blanchiment à base d'oxygène, 1 à 10 %, de préférence 2 à 6 % en poids d'activateur de blanchiment, caractérisé en ce qu'il contient 0,1 à 10 % en poids, en particulier 0,5 à 5 % en poids de granulé d'enzyme, dont le noyau comporte une enzyme et une matière porteuse inorganique et/ou organique, ainsi que le cas échéant un adjuvant de granulation et présente une couche d'enrobage uniforme, qui contient un inhibiteur de corrosion de l'argent.
